# FASCICULE DE BREVET EUROPEEN

(11) **EP 3 678 614 B1**
(45) Date de publication et mention de la délivrance du brevet: **14.01.2026**
(21) Numéro de dépôt: 18773811.7
(22) Date de dépôt: 05.09.2018
(51) Int. Cl.: A61F 5/01, A61F 5/34, A61F 5/32, A61F 5/03

(54) **DISPOSITIF DE RESTRICTION THORACIQUE ANTÉRO-POSTÉRIEUR**
VORRICHTUNG ZUR ANTEROPOSTERIOREN THORAKALEN RESTRIKTION
ANTEROPOSTERIOR THORACIC RESTRICTION DEVICE

(30) Priorité: 05.09.2017 FR 1758169
(43) Date de publication de la demande: 15.07.2020
(73) Titulaire: INSERM (Institut National de la Santé et de la Recherche Médicale), 75013 Paris (FR); Assistance Publique - Hôpitaux de Paris, 75004 Paris (FR); Université Paris-Est Créteil Val de Marne, 94010 Creteil (FR)
(72) Inventeur: MEKONTSO DESSAP, Armand, 75012 Paris (FR); CARTEAUX, Guillaume, 93100 Montreuil (FR)
(74) Mandataire: Cabinet Becker et Associés
(86) Numéro de dépôt international: PCT/FR2018/052163
(87) Numéro de publication internationale: WO 2019/048774

(56) Documents cités:
- FR-A- 1 006 109
- US-A- 3 454 000
- US-A- 4 928 674
- US-A1- 2003 004 445
- US-A1- 2007 272 250

## Description

L'invention a trait à un dispositif de restriction thoracique antéro-postérieur, destiné à entourer la cage thoracique d'un patient pour minimiser la ventilation en pression positive dans la partie antérieure des poumons dudit patient et favoriser la redistribution de la ventilation en pression positive vers la partie postérieure. L'invention trouve des applications dans le domaine médical, et notamment dans le domaine de l'assistance ventilatoire de patients présentant une pathologie respiratoire associée à des lésions pulmonaires inhomogènes. L'invention est particulièrement adaptée pour le traitement ou la prévention de l'insuffisance respiratoire aigüe ou chronique.

L'assistance ventilatoire par pression positive intra-thoracique est classiquement utilisée pour palier une insuffisance respiratoire d'un patient. Une interface respiratoire, invasive telle qu'une sonde d'intubation, ou non invasive, telle qu'un masque bucco-nasal ou facial, est alors appliquée sur le patient, et est connectée à un générateur de pression (appelé communément ventilateur) pour insuffler de manière artificielle de l'air dans les poumons. Cette délivrance globale de l'assistance ventilatoire est cependant inadaptée à la répartition de l'atteinte lésionnelle pulmonaire. En effet, la répartition des lésions pulmonaires est en règle générale inhomogène : il existe typiquement des zones condensées non aérées à répartition généralement postérieure, et des zones saines aérées antérieures. Cette répartition, particulièrement observée au cours du syndrome de détresse respiratoire aiguë (SDRA), s' explique notamment par le poids du cœur, la compression du poumon sus-jacent due à la gravité, et l'inhomogénéité naturelle de la compliance de la paroi thoracique, plus importante dans la partie antérieure que dans la partie postérieure, et ce d'autant plus que le patient est en décubitus dorsal. Ainsi, lorsque l'assistance ventilatoire est délivrée de manière globale, la ventilation se distribue préférentiellement dans les zones déjà aérées. Ceci expose à un déficit de ventilation des zones condensées (alors exposées à des lésions dites d'ouverture-fermeture), et un excès de ventilation des zones aérées (alors exposées à des lésions dites de sur-distension). Les lésions d'ouverture-fermeture et de sur-distension aggravent les lésions pulmonaires préexistantes et entrainent une surmortalité chez les patients.

Chez les patients présentant un SDRA, la position en décubitus ventral (allongé sur le ventre) tend à ré-homogénéiser la ventilation par différents effets physiologiques, en particulier la réduction de la compliance de la paroi thoracique antérieure. Il a été démontré que la position en décubitus ventral du patient pendant au moins 16 heures par 24 heures diminuait la mortalité au cours du SDRA. Cependant, le décubitus ventral est très peu pratiqué chez les patients sous ventilation artificielle présentant un SDRA, du fait de la lourdeur de la technique, de la fréquence des défaillances d'organes associées qui limitent sa faisabilité, des effets secondaires observés (escarres de la face, notamment).

Il n'existe pas à ce jour de dispositif permettant de limiter la sur-distension des zones correctement aérées des poumons et de favoriser la redistribution de la ventilation vers les zones condensées chez un patient nécessitant une assistance ventilatoire artificielle.

L'invention a pour objectif de résoudre au moins partiellement le problème associé à la répartition excessive de la ventilation au niveau des zones aérées des poumons au détriment des zones condensées chez un patient en décubitus dorsal ayant besoin d'une assistance ventilatoire artificielle. Pour cela, l'invention propose un dispositif destiné à être appliqué contre la cage thoracique du patient en décubitus dorsal, permettant de maximiser l'assistance ventilatoire dans les zones postérieures des poumons et de la minimiser dans les zones antérieures. Plus précisément, le dispositif selon l'invention comprend une interface de compression telle qu'une poche de fluide destinée à être maintenue en contact serré en regard du sternum du patient, de manière à appliquer une pression extra-thoracique positive localisée sur la partie antérieure de la paroi thoracique, en regard des zones pulmonaires généralement correctement aérées et donc exposées à la sur-distension. Ce dispositif permet de réduire spécifiquement la compliance de la paroi thoracique antérieure, donc d'homogénéiser la pression transpulmonaire et de favoriser ainsi la sollicitation de la partie postérieure des poumons. Des moyens de serrage bilatéraux permettent d'appliquer une pression mesurée dans la poche de fluide située en regard du sternum. Selon un autre aspect, le dispositif selon l'invention comprend au moins un moyen de serrage permettant d'appliquer une pression mesurée dans la poche de fluide située en regard du sternum.

L'invention a donc pour objet un dispositif de restriction thoracique antéro-postérieure comprenant au moins un moyen de maintien destiné à entourer la cage thoracique d'un patient, au moins une poche de fluide compressible, destinée à être maintenue contre le sternum du patient par au moins un moyen de maintien, et des moyens de serrage bilatéraux réversibles, disposés de part et d'autre de la poche de fluide et aptes à resserrer de manière réversible au moins un moyen de maintien autour de la cage thoracique du patient.

Dans le contexte de l'invention, le terme « patient » désigne un mammifère, et préférentiellement un humain, y compris un adulte, un enfant ou un nourrisson. Le terme « patient » peut également désigner un animal non humain, en particulier un primate non humain.

Selon l'invention, le ou les moyens de maintien permettent de maintenir la poche de fluide compressible contre le sternum du patient. En outre, lesdits moyens de maintien doivent présenter une faible compliance de manière à appliquer une pression homogène sur la poche de fluide.

Les moyens de serrage bilatéraux permettent de serrer de manière égale et contrôlée le ou les moyens de maintien autour de la cage thoracique du patient. Ainsi, la pression exercée est homogène de part et d'autre de la cage thoracique. Bien entendu, si cela s'avère nécessaire, il est possible de serrer de manières différentes les moyens de serrage bilatéraux, de manière à jouer sur la pression exercée de part et d'autre de la cage thoracique.

Dans un mode de réalisation les moyens de maintien comprennent une sangle, préférentiellement une sangle semi-rigide.

De manière alternative ou additionnelle, les moyens de maintien comprennent une plaque antérieure rigide ou semi rigide destinée à être appliquée contre la partie antérieure de la cage thoracique du patient, et optionnellement une plaque postérieure rigide ou semi rigide destinée à être appliquée contre la partie postérieure de la cage thoracique du patient. La plaque antérieure et/ou postérieure peut être multi-perforée afin d'adapter la position des moyens de serrage bilatéraux au diamètre du thorax du patient.

La poche de fluide est alors comprimée entre la plaque antérieure et la paroi thoracique du patient.

Dans un mode de réalisation, la plaque antérieure est en un seul tenant. Dans un autre mode de réalisation, la plaque antérieure comprend plusieurs éléments superposés les uns sur les autres.

Il est alors possible d'ajuster le nombre d'éléments pour jouer sur l'épaisseur de la plaque antérieure.

Dans un mode de réalisation, les moyens de maintien comprennent en outre des moyens de maintien latéraux ou pattes latérales destinés à relier la plaque antérieure aux moyens de serrage. Ces moyens de maintien latéraux peuvent être souples, rigides ou semi-rigides et s'étendent avantageusement de part et d'autre de la plaque antérieure. De manière préférée, les moyens de maintien latéraux sont disposés dans le prolongement de la plaque antérieure et sont éventuellement surélevés par rapport à la plaque antérieure. Dans un mode de réalisation, des renforts sont prévus afin de rigidifier le système des pattes latérales. Lesdits renforts peuvent comprendre une section en forme de L ou de T. De préférence, les bords longitudinaux des pattes latérales peuvent être profilés de sorte que leurs sections transversales soient en L ou en T afin de renforcer la rigidité des pattes latérales dans le sens longitudinal.

Avantageusement, les pattes latérales sont semi-rigides ou rigides, afin de transmettre de manière optimale la pression exercée par les moyens de serrage bilatéraux. Préférentiellement, les pattes latérales sont en outre surélevées par rapport à la plaque antérieure, de sorte qu'elles ne viennent pas en contact avec la peau du patient lorsque le dispositif est mis en place, et ce quels que soient le morphotype et le sexe du patient.

Dans un mode de réalisation, les dimensions de la plaque antérieure sont sensiblement égales à celles de la poche de fluide, de sorte que ladite plaque recouvre seulement ladite poche. Ce mode de réalisation permet de conserver un accès au patient pour le monitorage (notamment lorsque des patchs reliés à un écran de monitorage sont collés sur la face antérieure du thorax du patient), pour l'examen clinique et en particulier l'auscultation. Il permet également de limiter le risque de contact de la plaque antérieure avec la peau du patient et donc le risque d'intolérance cutanée et d'escarre.

Dans un autre mode de réalisation, les dimensions de la plaque antérieure sont strictement supérieures à celles de la poche de fluide compressible de sorte que la plaque s'étend de part et d'autre de la poche, de manière à recouvrir au moins partiellement la cage thoracique du patient.

Avantageusement, les moyens de serrage sont des moyens de serrage progressifs, de manière à contrôler et adapter plus précisément la pression appliquée sur la partie antéro-postérieure de la cage thoracique. Dans un mode de réalisation, les moyens de serrage sont antéro-latéraux.

Préférentiellement, le dispositif comprend également des moyens de desserrage automatique, de manière à pouvoir libérer rapidement et sans effort la cage thoracique du patient. Selon un autre mode de réalisation et lors de l'utilisation d'un moyen de serrage, le dispositif peut comprendre un seul moyen de desserrage automatique.

Avantageusement, le(s) moyen(s) de serrage sont aptes à appliquer une pression comprise entre 20 et 150 cm d'eau (cmH₂O), préférentiellement de 60 cmH₂O, +/- 20 dans la poche de fluide, lorsque celle-ci est maintenue entre le moyen de maintien et la cage thoracique d'un patient.

Dans un mode de réalisation, le dispositif de restriction thoracique antéro-postérieure comprend en outre un capteur de pression pour mesurer la pression dans la poche de fluide. Il est alors possible de contrôler précisément la pression extrathoracique appliquée.

Dans un mode de réalisation, la poche de fluide contient un liquide, préférentiellement de l'eau. Bien entendu, il est possible d'utiliser un autre fluide, et notamment un gaz.

Avantageusement, la quantité et/ou le volume de fluide contenu(s) dans la poche est constant. Par « constant » on entend que la quantité et/ou volume de fluide dans la poche à température et pression constantes est fixe, i.e. ne varie pas, préférentiellement à température ambiante (i.e., 20 à 30°C) et à pression atmosphérique. La poche contient une quantité définie et constante de fluide, au moins pendant toute la durée de l'utilisation du dispositif de restriction thoracique. Ainsi, la compression thoracique du patient résulte de la mise en œuvre des moyens de serrage et non des variations de la quantité et/ou du volume de fluide contenu dans la poche compressible. Préférentiellement, le volume de fluide constant contenu dans la poche est d'environ 1 litre ou 10⁶ mm³.

La taille de la poche de fluide est sensiblement égale aux dimensions du sternum du patient. D'une manière générale, les dimensions de la poche de fluide compressible sont telles qu'elle ne peut pas s'étendre sur les parties latérales et dorsale de la cage thoracique.

Dans un mode de réalisation, les moyens de maintien comprennent un logement central destiné à recevoir la poche de fluide. Par exemple, les moyens de maintien comprennent une sangle destinée à entourer la cage thoracique et un logement est ménagé dans la partie antérieure interne de la sangle, destinée à être appliquée contre la paroi thoracique. Dans un autre exemple, les moyens de maintien comprennent une plaque antérieure destinée à comprimer la cage thoracique antérieure et un logement est ménagé sur la face interne de la plaque, destinée à être appliquée contre la paroi thoracique.

L'invention a également pour objet un système de ventilation artificielle comprenant un ventilateur relié à une interface nasale et/ou buccale et/ou trachéale, pour amener de l'air dans les poumons d'un patient, ledit système comprenant en outre un dispositif de restriction thoracique antéro-postérieure selon l'invention.

L'invention a aussi pour objet un kit de ventilation artificielle comprenant un tel système de ventilation artificielle, et des moyens pour appliquer une dépression sur au moins une partie de la cage thoracique du patient. Documents FR 1 006 109 A et US 2003/004445 A1 décrivent des dispositifs de restriction thoracique.

L'invention sera mieux comprise à la lecture de la description qui suit et à l'examen des figures qui l'accompagnent. Celles-ci sont présentées à titre indicatif et nullement limitatif de l'invention. Les figures représentent :
Figure 1 : une représentation schématique en coupe transversale de la cage thoracique d'un patient présentant des lésions inhomogènes, avec condensation de la partie postérieure des poumons droit et gauche ;
Figure 2 : une représentation schématique en coupe transversale de la cage thoracique d'un patient sur lequel est maintenu un dispositif de restriction thoracique antéro-postérieur selon un premier exemple de réalisation de l'invention ;
Figure 3 : une représentation schématique en coupe transversale de la cage thoracique d'un patient sur lequel est maintenu un dispositif de restriction thoracique antéro-postérieur selon un second exemple de réalisation de l'invention ;
Figure 4 : une représentation schématique en coupe transversale de la cage thoracique d'un patient sur lequel est maintenu un dispositif de restriction thoracique antéro-postérieur selon un troisième exemple de réalisation de l'invention ;
Figure 5 : une représentation schématique de la distribution essentiellement antérieure de la ventilation artificielle en pression positive chez un patient ne bénéficiant pas du dispositif selon l'invention (A) et de la redistribution postérieure et inférieure de la ventilation artificielle en pression positive chez un patient muni du dispositif selon l'invention (B).
Figure 6 : une représentation schématique en coupe transversale de la cage thoracique d'un patient sur lequel est maintenu un dispositif de restriction thoracique antéro-postérieur selon un quatrième exemple de réalisation de l'invention ;
Figure 7 : une représentation schématique d'un dispositif de restriction thoracique selon un exemple de réalisation de l'invention.
Figure 8 : une copie d'écran d'un dispositif d'électroimpédancemétrie thoracique (EIT) d'un cadavre (modèle Thiel) ventilé mécaniquement comprenant le dispositif selon la figure 4. Le cadre 1 décrit des enregistrements dynamiques d'EIT du modèle Thiel. Le cadre 2 décrit les modifications d'impédance globale et les modifications d'impédance par région d'intérêt, depuis la région la plus antérieure (ROI 1) jusqu'à la région la plus postérieure (ROI 4), en fonction du temps. Le cadre 3 décrit le pourcentage de la ventilation totale qui atteint une région d'intérêt.

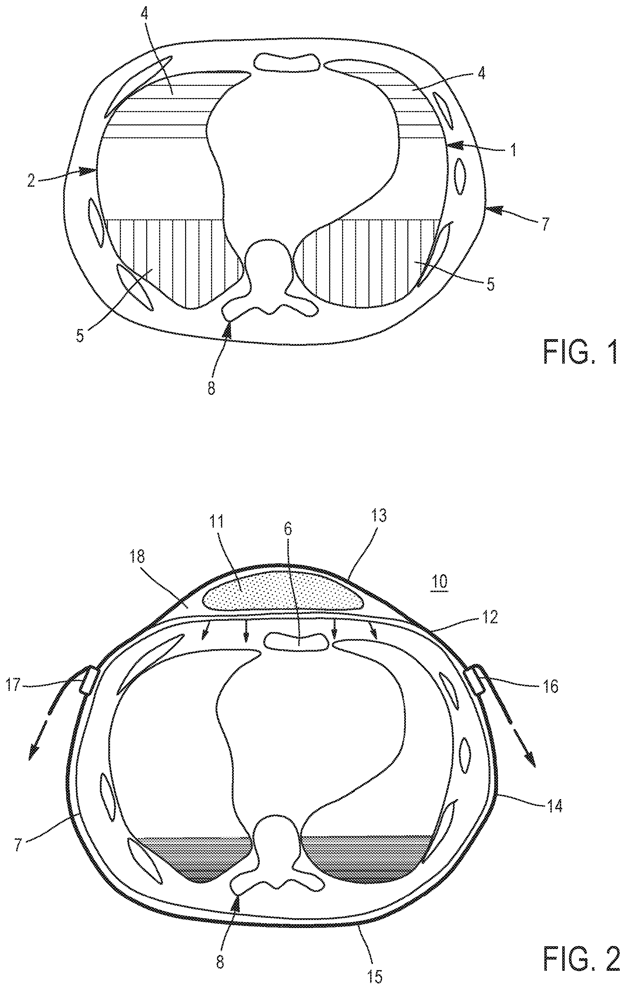

Comme exposé ci-dessus, certains patients en insuffisance respiratoire présentent des lésions inhomogènes des poumons 1, 3. La figure 1 représente une coupe transversale d'une cage thoracique d'un patient humain présentant la répartition inhomogène classique des lésions pulmonaires, allongé sur le dos (en décubitus dorsal), vertèbres 8 vers le bas sur la figure. La répartition de la ventilation globale en pression positive entraine une sur-distension des zones antérieures 4 des poumons 1, 2 et une absence de ventilation ou une mauvaise ventilation des zones condensées postérieures 5 desdits poumons, exposées aux lésions d'ouverture-fermeture.

Le dispositif selon l'invention permet de pallier ce problème en limitant la ventilation dans la partie antérieure aérée des poumons et en favorisant la mobilisation de la partie postérieure des poumons. Plus précisément, le dispositif selon l'invention permet d'appliquer une pression positive extrathoracique au niveau de la partie antérieure de la cage thoracique au niveau de laquelle ledit dispositif est appliqué, entrainant une diminution régionale de la pression transpulmonaire. Cette diminution régionale de la pression transpulmonaire à l'endroit où elle était la plus élevée tend en fait à homogénéiser la pression transpulmonaire au niveau de l'ensemble des poumons. Lorsqu'une ventilation artificielle par pression positive est administrée simultanément au patient, les zones pulmonaires antérieures aérées sont protégées des lésions de sur-distension par la diminution régionale de la pression transpulmonaire, et la ventilation tend à se redistribuer vers les régions postérieures du fait de l'homogénéisation de la pression transpulmonaire, ce d'autant que le mode de ventilation est en volume contrôlé (c'est-à-dire insufflation d'un volume courant pré-réglé à une fréquence imposée, jusqu'à atteinte du volume fixé, sans participation du patient et sans tenir compte de son activité respiratoire).

Les figures 2 à 4 et 6 représentent de manière schématique des coupes transversales d'une cage thoracique d'un patient humain, en décubitus dorsal, muni de différents modes de réalisation du dispositif de restriction thoracique antéro-postérieure selon l'invention, propre à limiter la sur-distension des zones antérieures et à favoriser la mobilisation postérieure des poumons lors d'une ventilation artificielle en pression positive.

Sur la figure 2, le dispositif de restriction thoracique antéro-postérieure 10 comprend une poche de fluide 11 maintenue contre le sternum 6 du patient au moyen d'une sangle 12, formant moyens de maintien, qui entoure la cage thoracique 7 dudit patient. La sangle 12 est en matériau rigide ou semi-rigide, de manière à ce que la faible compliance de ces moyens de maintien permette une pression d'application de la poche 11 suffisante contre le sternum 6 du patient. Dans un mode de réalisation, la sangle 12 est en un matériau biocompatible, tel que du polyuréthane. Par « matériau biocompatible », on entend un matériau apte à être utilisé dans ou sur des tissus biologiques, sans dégrader les tissus biologiques concernés ou déclencher de réactions allergiques pendant ou après le contact. Dans le cadre de l'invention, le matériau biocompatible utilisé doit notamment tenir compte des propriétés de la peau du patient. Dans un autre mode de réalisation, la sangle 12 est en cuir. Il est possible d'utiliser une sangle d'épaisseurs variables, et notamment une sangle présentant une épaisseur plus importante au niveau de la zone de contact 13 avec la poche de fluide 11, qu'au niveau des parties antéro-latérales 14 et postérieure 15.

Des moyens de serrage 16, 17 bilatéraux indépendants, disposés aux niveaux antéro-latéraux de la cage thoracique 7, permettent de resserrer la sangle 12 autour de la cage thoracique 7 du patient, de manière à augmenter la pression positive extrathoracique sur le sternum 6 par le biais de la poche de fluide 11.

Avantageusement, la poche de fluide 11 est maintenue en position sur la sangle 12, de manière à ne pas être déplacée en cours d'utilisation. Par exemple, comme cela est représenté sur la figure 2, la sangle 12 comprend un logement 18 dans lequel la poche de fluide 11 peut être introduite et maintenue.

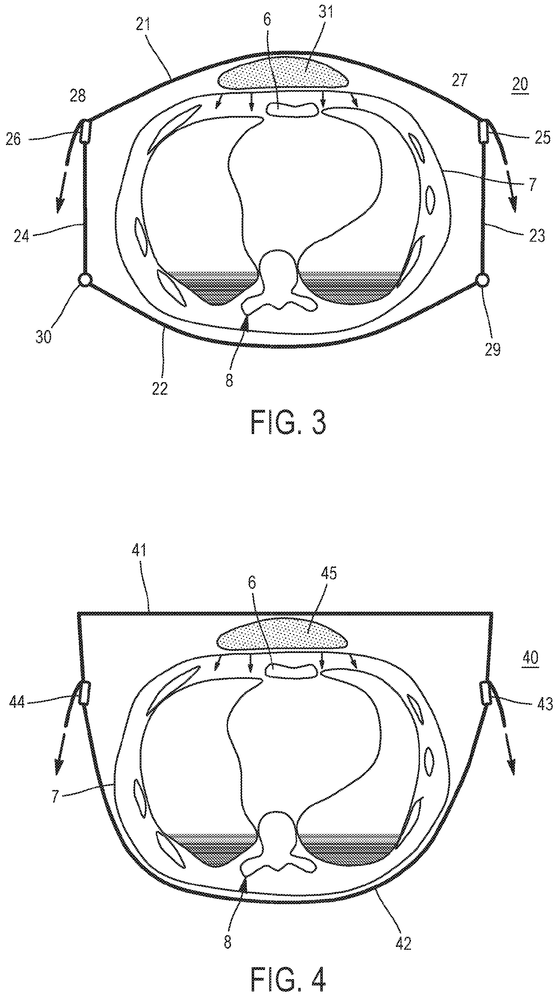

Dans l'exemple de réalisation représenté à la figure 3, le dispositif de restriction thoracique antéro-postérieure 20 comprend deux plastrons semi-rigides en matériau préférentiellement biocompatible, respectivement antérieur 21 et postérieur 22, formant les moyens de maintien. Le plastron antérieur 21 est appliqué contre la partie antérieure de la cage thoracique du patient, tandis que le plastron postérieur 22 est appliqué contre la partie postérieure de la cage thoracique du patient. Des moyens de serrage permettent de maintenir les moyens de maintien en position sur la cage thoracique du patient. Plus précisément, les moyens de serrage comprennent deux sangles de serrage 23, 24, chacune associée à des systèmes de serrage indépendants 25, 26. Les sangles de serrage 23, 24 permettent chacune de relier une extrémité latérale 27, 28, d'un premier plastron 21 à une extrémité latérale 29, 30, du second plastron 22. Une poche de fluide 31 est comprimée entre le plastron antérieur 21 et le sternum 6 de la cage thoracique 7 du patient. Bien entendu, il est possible d'utiliser un dispositif de restriction thoracique ne comprenant que le plastron antérieur. Dans ce cas, les moyens de serrage comprennent une sangle de serrage qui entoure entièrement la partie postérieure et les parties latérales de la cage thoracique du patient.

Dans l'exemple de réalisation représenté à la figure 4, le dispositif de restriction thoracique antéro-postérieure 40 comprend une plaque antérieure 41 rigide en matériau préférentiellement biocompatible. Les moyens de serrage, eux, comprennent une sangle de serrage 42 entourant entièrement les parties antéro-latérales et postérieures de la cage thoracique 7 du patient. Les moyens de serrage comprennent également des systèmes de serrage bilatéraux 43, 44 indépendants. Une poche de fluide 45 est comprimée entre la plaque antérieure 41 et le sternum 6 de la cage thoracique 7 du patient.

Dans un mode de réalisation, les moyens de maintien comprennent des bretelles, préférentiellement réglables en hauteur. Les bretelles sont, par exemple, fixées en haut de la sangle 12, du ou des plastrons 21, 22 ou de la plaque antérieure 41. Les bretelles permettent d'éviter un glissement des moyens de maintien et de la poche de fluide vers le bas du corps du patient, et participent ainsi au maintien de la poche de fluide en position sur le sternum. Les bretelles peuvent consister en, ou comporter, des bandes textiles à boucles et crochets (telles que des bandes Velcro^{®}), aisément adaptables en longueur et repositionnables.

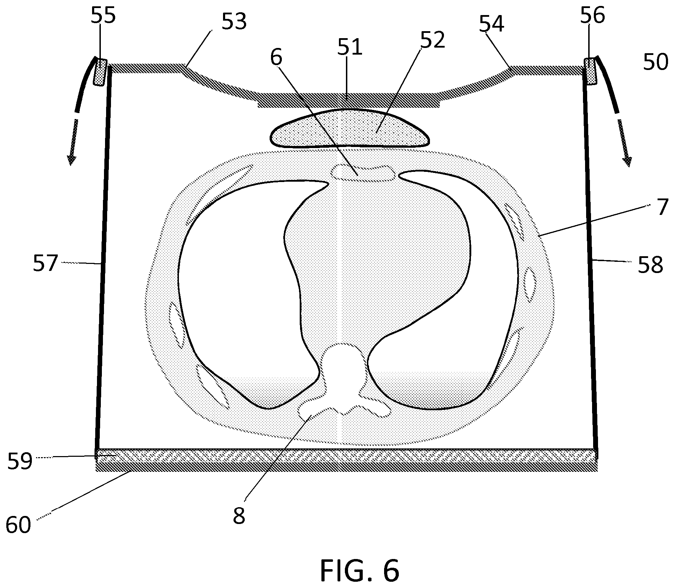

Dans l'exemple de réalisation représenté à la figure 6, le dispositif de restriction thoracique antéro-postérieure 50 comprend une plaque antérieure 51 rigide ou semi-rigide en matériau préférentiellement biocompatible, par exemple un plastique biocompatible. Une poche de fluide 52 est comprimée entre la plaque antérieure 51 et le sternum 6 de la cage thoracique 7 du patient. La plaque antérieure 51 est reliée aux moyens de serrage par des moyens de maintien latéraux 53, 54.

Les moyens de maintien latéraux 53, 54 peuvent être en matériau semi-rigide ou rigide, tel qu'un plastique ou un métal tel que l'aluminium. Dans le cas où ils sont en matériau semi-rigide, il est possible de prévoir des renforts permettant de les rigidifier davantage.

Les moyens de serrage comprennent des sangles de serrage 57, 58 reliant les moyens de maintien latéraux à un point d'ancrage postérieur sans entrer en contact avec la cage thoracique 7 du patient. Les moyens de serrage comprennent également des systèmes de serrage bilatéraux 55, 56 indépendants.

Dans l'exemple représenté figure 6, les moyens de serrage sont reliés à une plaque postérieure 60 rigide ou semi-rigide maintenue contre la partie dorsale de la cage thoracique du patient. Cette plaque postérieure 60 est avantageusement recouverte sur sa face destinée à être en contact avec la peau du patient d'un matériau visant à optimiser la tolérance cutanée et limiter le risque d'escarre, par exemple un gel viscoélastique. De préférence, la plaque postérieure 60 est multi-perforée afin d'adapter le point d'ancrage des sangles de serrage 57,58 aux dimensions de la partie dorsale du thorax du patient. Selon un autre mode de réalisation, la plaque postérieure forme une coque postérieure épousant une partie de la paroi thoracique postéro-latérale. Avantageusement les moyens de serrages sont fixés de part et d'autre de la coque postérieure, par exemple par des anneaux. Bien entendu, il est possible d'utiliser ce dispositif de restriction thoracique sans plaque postérieure. Dans ce cas, une sangle de serrage unique entoure avantageusement les parties antéro-latérales et dorsales de la cage thoracique du patient, comme illustré dans les figures 3 et 4.

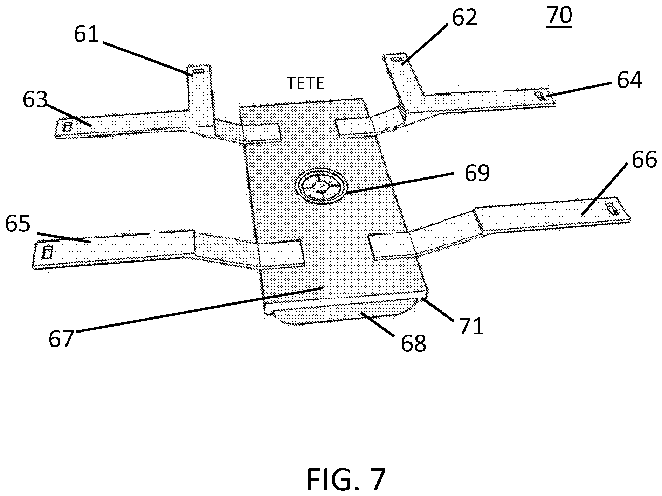

Dans l'exemple de réalisation représenté à la figure 7, le dispositif de restriction thoracique antéro-postérieure 70 comprend une plaque antérieure 67 rigide ou semi-rigide en matériau préférentiellement biocompatible. Une poche de fluide 68 destinée à être appliquée sur le sternum de la cage thoracique du patient est fixée sur la paroi interne (71) de la paque antérieure 67. Cette fixation peut être réversible ou irréversible.

La plaque antérieure 67 peut intégrer un capteur de pression 69 en communication directe avec la poche 68. Ce capteur de pression 69 permet de visualiser directement la pression à l'intérieur la poche de fluide 68.

La plaque antérieure comprend des moyens de maintien latéraux 63, 64, 65, 66, fixés à ladite plaque. De préférence, ces moyens de maintien latéraux comprennent des renforts permettant de les rigidifier. Selon un mode de réalisation, les moyens de maintien latéraux 63, 64 comprennent des moyens d'attache 61, 62 destinés à fixer des bretelles ou sangles pour maintenir le dispositif dans le sens cranio-caudal.

Dans l'exemple illustré à la figure 7, les renforts des moyens de maintien latéraux 63, 64, destinés à être les plus proches des épaules du patient, sont en forme de L, afin de ménager des moyens de fixation à des bretelles.

Les moyens de maintien latéraux 63, 64, 65, 66 s'étendent en saillie de la plaque antérieure 67, de part et d'autre de ladite plaque de manière à se prolonger perpendiculairement à la cage thoracique du patient. Dans l'exemple représenté à la figure 7, les moyens de maintien latéraux63, 64, 65, 66 sont surélevés par rapport à la plaque antérieure.

Les moyens de maintien et/ou renforts comprennent avantageusement des orifices, destinés à l'introduction de moyen de serrage.

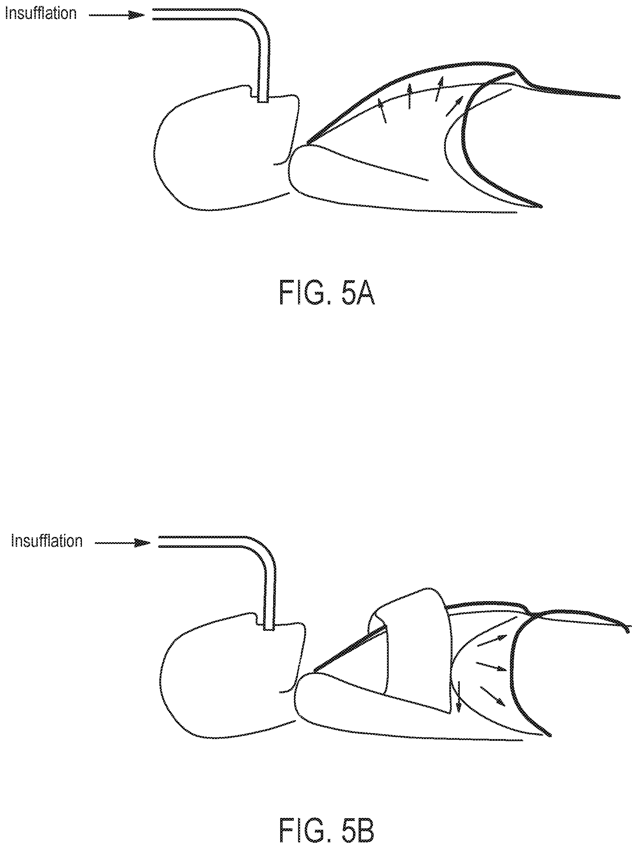

D'une manière générale, le dispositif selon l'invention permet lorsqu'il est utilisé en même temps qu'un système de ventilation artificielle à pression positive (invasif ou non invasif) de limiter la compliance de la paroi thoracique antérieure et ainsi de limiter le risque de sur-distension des zones antérieures et favoriser la redistribution de l'air insufflé dans les zones postérieures et inférieures des poumons (figure 5B). A l'inverse, en l'absence d'un tel dispositif (figure 5A), la distribution est essentiellement antérieure, les zones postérieures et inférieures étant peu ou pas ventilées.

### Preuve de concept

La preuve de concept a été réalisée sur un modèle cadavérique Thiel (« cadavre Thiel ») sous ventilation mécanique invasive. Les cadavres Thiel ont bénéficié d'une méthode d'embaumement particulière, leur permettant de conserver l'élasticité naturelle des tissus. De manière particulièrement intéressante, les poumons des cadavres Thiel sous ventilation mécanique se comportent de manière identique aux poumons d'un patient atteint de SDRA, avec des zones postérieures condensées et des zones antérieures aérées. Les paramètres de mécanique respiratoire sont ainsi comparables à ceux d'un patient atteint de SDRA.

Au cours de l'étape de preuve de concept, un dispositif d'électroimpédancemétrie thoracique (EIT) a été appliqué à des cadavres munis d'un dispositif de restriction thoracique antéro-postérieure selon l'invention tel que décrit à la figure 4, ou non munis d'un tel dispositif. L'EIT permet de mesurer régionalement des modifications d'impédance, qui correspondent à des modifications d'aération au cours de la ventilation mécanique invasive. L'EIT permet donc de visualiser directement les zones ventilées et de quantifier régionalement le gain ou la perte d'aération après une intervention.

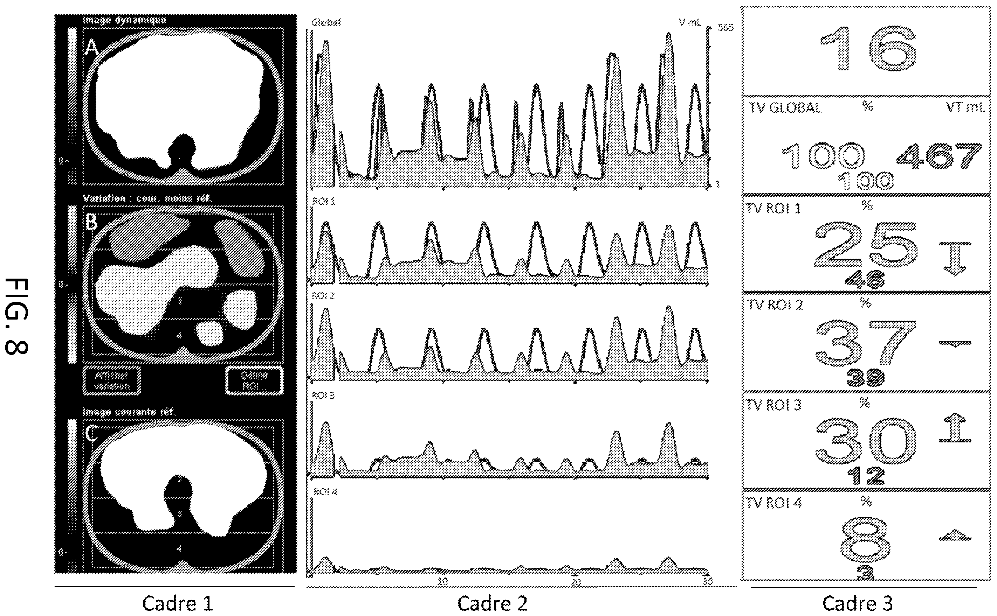

La figure 8 décrit une copie d'écran de l'EIT d'un cadavre ventilé mécaniquement sur lequel le dispositif selon l'invention a été appliqué.

Le cadre 1 représente des enregistrements dynamiques d'EIT du cadavre au cours du schéma expérimental. L'EIT rapporte des modifications régionales d'impédance sur une coupe transversale du thorax. Pour chaque coupe, le dos est en bas et la partie antérieure du thorax est en haut. Sur la coupe (C) apparait en blanc l'enveloppe de l'ensemble des régions pulmonaires ventilées au cours de l'insufflation du ventilateur sans le dispositif selon l'invention. Sur la coupe (A) apparait en blanc l'enveloppe de l'ensemble des régions pulmonaires ventilées au cours de l'insufflation du ventilateur avec le dispositif selon l'invention en place (pression appliquée sur la paroi thoracique antérieure : environ 80 cm H2O). La coupe (B) représente les différences régionales d'aération entre l'étape expérimentale de la coupe (C), c'est-à-dire sans le dispositif selon l'invention, et l'étape expérimentale de la coupe (A), c'est-à-dire avec le dispositif selon l'invention. Les régions en gris foncé correspondent à des diminutions d'aération entre l'étape (C) et l'étape (A). Les régions en gris clair correspondent à des gains d'aération entre l'étape (C) et l'étape (A). On peut observer que l'application du dispositif selon l'invention entraine une diminution de l'aération des zones antérieures (qui restent cependant ventilées comme en témoigne l'enveloppe des zones ventilées sur la coupe (A)) et une augmentation de l'aération des zones postérieures avec un gain du volume total du poumon ventilé, correspondant à un recrutement des zones postérieures précédemment non aérées.

Le cadre 2 représente les modifications d'impédance globale (courbe du haut) et les modifications d'impédance par région d'intérêt, depuis la région la plus antérieure (ROI 1) jusqu'à la région la plus postérieure (ROI 4), en fonction du temps, au cours de l'application du dispositif selon l'invention. Les quatre régions d'intérêt correspondent aux quatre rectangles numérotés de 1 à 4 sur les coupes d'EIT du cadre 1. Ces modifications d'impédance prises isolément sont difficiles d'interprétation et il faut se rapporter au cadre 3 pour en comprendre l'intérêt.

Le cadre 3 décrit les proportions régionales de modifications d'impédance par rapport à la modification d'impédance globale. Il s'agit donc du pourcentage de la ventilation totale qui atteint une région d'intérêt. Dans chaque région d'intérêt, le chiffre en grands caractères rapporte le pourcentage de la ventilation totale avec le dispositif selon l'invention en place ; le chiffre en dessous, en petits caractères, rapporte le pourcentage de la ventilation totale sans le dispositif selon l'invention.

Ainsi, en l'absence de dispositif selon l'invention, 85% de la ventilation totale se répartit dans la moitié antérieure du thorax (ROI 1 et 2) et 15% seulement dans la moitié postérieure. Avec l'application du dispositif selon l'invention, 62% de la ventilation totale se répartit dans la moitié antérieure et 38% dans la moitié postérieure.

Les résultats décrits ci-dessus montrent donc que l'utilisation du dispositif selon l'invention permet une diminution de la ventilation dans les zones antérieures limitant le risque de sur-distension, au bénéfice d'un gain d'aération dans les zones postérieures.

## Revendications

1. Dispositif de restriction thoracique antéro-postérieure (10, 20, 40, 50, 70) comprenant des moyens de maintien destinés à entourer la cage thoracique d'un patient, une poche de fluide (11) compressible comprenant une quantité de fluide constant, destinée à être maintenue contre le sternum du patient par lesdits moyens de maintien, et des moyens de serrage bilatéraux (16, 17 ; 25, 26 ; 43, 44) réversibles, disposés de part et d'autre de la poche de fluide et aptes à resserrer de manière réversible les moyens de maintien autour de la cage thoracique du patient, **caractérisé en ce que** la taille de la poche est sensiblement égale aux dimensions du sternum du patient.

2. Dispositif de restriction thoracique antéro-postérieure selon la revendication 1, dans lequel les moyens de maintien comprennent une plaque antérieure (21, 41) rigide ou semi rigide destinée à être appliquée contre la partie antérieure de la cage thoracique du patient, et optionnellement une plaque postérieure (22) rigide ou semi rigide destinée à être appliquée contre la partie postérieure de la cage thoracique du patient.

3. Dispositif de restriction thoracique antéro-postérieure selon la revendication 1 ou 2, dans lequel les moyens de maintien comprennent une sangle (14), préférentiellement une sangle semi-rigide.

4. Dispositif de restriction thoracique antéro-postérieure selon l'une des revendications précédentes, dans lequel les moyens de serrage sont des moyens de serrage progressifs.

5. Dispositif de restriction thoracique antéro-postérieure selon l'une des revendications précédentes, dans lequel les moyens de serrage sont antéro-latéraux et/ou dans lequel les moyens de serrage sont aptes à appliquer une pression comprise entre 20 et 150 cm d'eau (cmH₂O), préférentiellement de 60 cmH2O, +/- 20 dans la poche de fluide, lorsque celle-ci est maintenue entre la sangle et la cage thoracique d'un patient.

6. Dispositif de restriction thoracique antéro-postérieure selon l'une des revendications précédentes, ledit dispositif comprenant en outre un capteur de pression pour mesurer la pression dans la poche de fluide et/ou des moyens de desserrage automatique.

7. Dispositif de restriction thoracique antéro-postérieure selon l'une des revendications précédentes, dans lequel la poche de fluide contient un liquide, préférentiellement de l'eau.

8. Dispositif de restriction thoracique antéro-postérieure selon la revendication 7, dans lequel le volume dudit liquide est constant dans la poche de fluide compressible.

9. Dispositif de restriction thoracique antéro-postérieure selon l'une des revendications précédentes, dans lequel la sangle ou plaque antérieure comprend un logement central (18, 69) destiné à recevoir la poche de fluide.

10. Système de ventilation artificielle comprenant un ventilateur relié à une interface nasale et/ou buccale et/ou trachéale, pour amener de l'air dans les poumons d'un patient, ledit système comprenant en outre un dispositif de restriction thoracique antéro-postérieure selon l'une des revendications 1 à 9.

11. Kit de ventilation artificielle comprenant un système de ventilation artificielle selon la revendication 10, et des moyens pour appliquer une dépression sur au moins une partie de la cage thoracique du patient.

## Patentansprüche

1. Anteroposteriore Thorax-Restriktionsvorrichtung (10, 20, 40, 50, 70) enthaltend Fixierungselemente zur Umschließung des Brustkorbs eines Patienten, einen komprimierbaren Flüssigkeitsbeutel (11) mit konstanter Flüssigkeitsmenge, der durch diese Fixierungselemente gegen das Brustbein des Patienten gedrückt werden soll, und reversible, beidseitige Spannvorrichtungen (16, 17; 25, 26; 43, 44), die beidseitig des Flüssigkeitsbeutels angeordnet sind und die Fixierungselemente reversibel um den Brustkorb des Patienten festziehen können, **dadurch gekennzeichnet, dass** die Größe des Beutels im Wesentlichen den Abmessungen des Brustbeins des Patienten entspricht.

2. Anteroposteriore Thorax-Restriktionsvorrichtung nach Anspruch 1, wobei die Fixierungselemente eine starre oder halbstarre vordere Platte (21, 41) umfassen, die dazu bestimmt sind, gegen den vorderen Teil des Brustkorbs des Patienten angelegt zu werden, und optional eine starre oder halbstarre hintere Platte (22), die dazu bestimmt ist, gegen den hinteren Teil des Brustkorbs des Patienten angelegt zu werden.

3. Anteroposteriore Thorax-Restriktionsvorrichtung nach Anspruch 1 oder 2, wobei die Fixierungseinrichtung einen Gurt (14), vorzugsweise einen halbstarren Gurt, umfasst.

4. Anteroposteriore Thorax-Restriktionsvorrichtung nach einem der vorhergehenden Ansprüche, wobei es sich bei den Spannvorrichtungen um progressive Spannvorrichtungen handelt.

5. Anteroposteriore Thorax-Restriktionsvorrichtung nach einem der vorhergehenden Ansprüche, wobei die Spannvorrichtungen anterolateral sind und/oder wobei die Spannvorrichtungen in der Lage sind, einen Druck zwischen 20 und 150 cm Wassersäule (cmH2O), vorzugsweise 60 cmH2O, +/- 20, im Flüssigkeitsbeutel auszuüben, wenn dieser zwischen dem Gurt und dem Brustkorb eines Patienten gehalten wird.

6. Anteroposteriore Thorax-Restriktionsvorrichtung nach einem der vorhergehenden Ansprüche, wobei diese Vorrichtung ferner einen Drucksensor zur Messung des Drucks im Flüssigkeitsbeutel und/oder Mittel zur automatischen Lockerung umfasst.

7. Anteroposteriore Thorax-Restriktionsvorrichtung nach einem der vorhergehenden Ansprüche, wobei der Flüssigkeitsbeutel eine Flüssigkeit, vorzugsweise Wasser, enthält.

8. Anteroposteriore Thorax-Restriktionsvorrichtung nach Anspruch 7, wobei das Volumen dieser Flüssigkeit in dem komprimierbaren Flüssigkeitsbeutel konstant ist.

9. Anteroposteriore Thorax-Restriktionsvorrichtung nach einem der vorhergehenden Ansprüche, wobei der vordere Gurt oder die vordere Platte ein zentrales Gehäuse (18, 69) aufweist, das zur Aufnahme des Flüssigkeitsbeutels bestimmt ist.

10. Künstliches Beatmungssystem enthaltend ein Beatmungsgerät, das mit einer nasalen und/oder oralen und/oder trachealen Schnittstelle verbunden ist, um Luft in die Lunge eines Patienten zu leiten, wobei dieses System ferner eine anteroposteriore Thorax-Restriktionsvorrichtung nach einem der Ansprüche 1 bis 9 umfasst.

11. Künstliches Beatmungskit, enthaltend ein künstliches Beatmungssystem nach Anspruch 10 und Elemente zum Anlegen eines Unterdrucks an mindestens einen Teil des Brustkorbs des Patienten.

## Claims

1. An anteroposterior thoracic restriction device (10, 20, 40, 50, 70) comprising holding means intended to surround the rib cage of a patient, a compressible fluid bag (11) comprising a constant amount of fluid, intended to be held against the patient's sternum by said holding means, and reversible bilateral tightening means (16, 17; 25, 26; 43, 44) that are disposed on either side of the fluid bag and are capable of reversibly tighten the holding means around the patient's rib cage, **characterised in that** the size of the bag is substantially equal to the dimensions of the patient's sternum.

2. The anteroposterior thoracic restriction device according to claim 1, wherein the holding means comprise a rigid or semi-rigid anterior plate (21, 41) intended to be applied against the anterior part of the patient's rib cage, and optionally a rigid or semi-rigid posterior plate (22) intended to be applied against the posterior part of the patient's rib cage.

3. The anteroposterior thoracic restriction device according to claim 1 or 2, wherein the holding means comprise a strap (14), preferably a semi-rigid strap.

4. The anteroposterior thoracic restriction device according to one of the preceding claims, wherein the tightening means are progressive tightening means.

5. The anteroposterior thoracic restriction device according to one of the preceding claims, wherein the tightening means are anterolateral tightening means and/or wherein the tightening means are capable of applying a pressure of between 20 and 150 cm of water (cmH₂O), preferably 60+/-20 cmH2O, in the fluid bag, when the latter is held between the strap and the rib cage of a patient.

6. The anteroposterior thoracic restriction device according to one of the preceding claims, said device further comprising a pressure sensor for measuring the pressure in the fluid bag and/or automatic release means.

7. The anteroposterior thoracic restriction device according to one of the preceding claims, wherein the fluid bag contains a liquid, preferably water.

8. The anteroposterior thoracic restriction device according to claim 7, wherein the volume of said liquid is constant in the compressible fluid bag.

9. The anteroposterior thoracic restriction device according to one of the preceding claims, wherein the strap or anterior plate comprises a central housing (18, 69) intended to receive the fluid bag.

10. An artificial ventilation system comprising a ventilator connected to a nasal and/or buccal and/or tracheal interface, for delivering air into the lungs of a patient, said system further comprising an anteroposterior thoracic restriction device according to one of claims 1 to 9.

11. An artificial ventilation kit comprising an artificial ventilation system according to claim 10, and means for applying a negative pressure to at least one part of the patient's rib cage.
